(19) Europäisches Patentamt
European Patent Office
Office européen des brevets

(11) Numéro de publication: **0 010 032**
**B1**

(12) **FASCICULE DE BREVET EUROPEEN**

(45) Date de publication du fascicule du brevet: **21.04.82**

(51) Int. Cl.³: **C 07 C 50/18**

(21) Numéro de dépôt: **79400672.6**

(22) Date de dépôt: **21.09.79**

(54) **Procédé amélioré pour la préparation de l'anthraquinone en milieu aqueux.**

(30) Priorité: **10.10.78 FR 7828829**

(43) Date de publication de la demande:
**16.04.80 Bulletin 80/8**

(45) Mention de la délivrance du brevet:
**21.04.82 Bulletin 82/16**

(84) Etats contractants désignés:
**BE CH DE FR GB IT NL**

(56) Documents cités:
**DE - A - 2 206 864**

(73) Titulaire: **P C U K PRODUITS CHIMIQUES UGINE KUHLMANN**
**Service Propriété Industrielle Tour Manhattan**
**F-92087 Paris La Defence 2 Cédex 21 (FR)**

(72) Inventeur: **Dubreux, Bernard**
**29, avenue du Charter Bât.A.3**
**F-69340 Francheville le Bas (FR)**
Inventeur: **Tellier, Pierre**
**106, avenue de la Libération**
**F-69110 Sainte foy les Lyon (FR)**
Inventeur: **Delavarenne, Serge Yvon**
**2, rue des Alouettes**
**F-69340 Francheville le Haut (FR)**

(74) Mandataire: **Houssin, Jean et al,**
**PRODUITS CHIMIQUES UGINE KUHLMANN**
**Service Propriété Industrielle Tour Manhattan**
**Cedex 21**
**F-92087 Paris La Defense 2 (FR)**

**EP 0 010 032 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé amélioré pour la préparation de l'anthraquinone en milieu aqueux

La présente invention concerne une amélioration au procédé de préparation de l'anthraquinone en milieu aqueux à partir du produit d'addition de la naphtoquinone et du butadiène.

L'anthraquinone et ses dérivés de substitution sont des intermédiaires importants en particulier dans la chimie des colorants. Depuis fort longtemps, on sait obtenir l'anthraquinone par oxydation directe de l'anthracène. Le développement des applications de l'anthraquinone parallèlement à la limitation de la capacité mondiale d'approvisionnement en anthracène contribuent toutefois à rendre compétitives d'autres voies d'accès à ce dérivé. C'est le cas de la voie mettant en jeu la naphtoquinone et le butadiène avec le schéma réactionnel, en deux étapes, suivant:

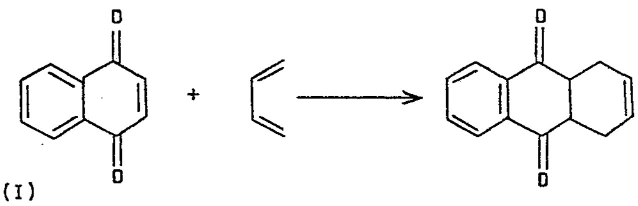

(I) (II)

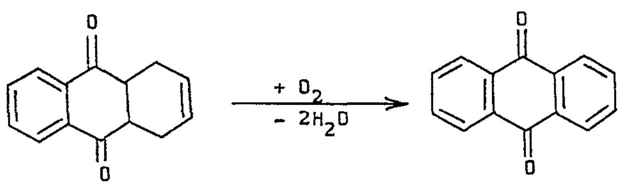


(II) (III)

Différentes techniques pour réaliser la seconde étape d'oxydation de la tétrahydro-1,4,4a,9a anthraquinone (II) en anthraquinone (III) sont connues depuis longtemps. De nombreuses solutions ont été proposées parmi lesquelles la plus simple à mettre en oeuvre est l'oxydation par insufflation d'air soit dans une suspension du précurseur (II) dans une solution aqueuse alcaline (brevet US 2.652.408 demandé le 14 Juillet 1951), soit dans une solution de ce même précurseur dans un solvant organique à caractère basique, une amine tertiaire par exemple (brevet belge 823.876 demandé le 27 Décembre 1974). Le fait d'opérer en solution aqueuse présente l'avantage de la simplicité et est par ailleurs plus économique car il permet d'éviter les pertes de solvant lors des manipulations ultérieures de récupération, de purification et de recyclage. Par contre, des phénomènes de moussage sont observés qui rendent technologiquement la mise en oeuvre industrielle de cette technique très difficile et entrainent une baisse importante de la productivité (brevet japonais, Kokaï 76.54.540 du 7 Novembre 1974).

La présente invention apporte une solution à ce problème. La demanderesse vient en effet de trouver que l'oxydation de la tétrahydro-1,4,4a,9a anthraquinone en anthraquinone peut être réalisée par bullage d'air dans une solution aqueuse alcaline de tétrahydro-1,4,4a,9a anthraquinone de manière simple et rapide, avec de très bons rendements et avec suppression des phénomènes de moussage, par adjonction au milieu de faibles quantités d'alcool.

Les alcools utilisables selon l'invention sont les alcools primaires ou secondaires aliphatiques monofonctionnels, linéaires ou ramifiés, comprenant 2 à 10 atomes de carbone.

Parmi les alcools utilisables selon l'invention on peut mentionner l'éthanol, le propanol normal, l'isopropanol, les butanols primaires et le butanol secondaire, les alcools amyliques primaires et secondaires, les octanols primaires ou secondaires, etc ...

Pour la mise en oeuvre du procédé on peut utiliser des solutions aqueuses de soude, de potasse, de chaux ou de carbonates de métaux alcalins à des concentrations pouvant varier entre 1% et la saturation. La concentration en poids de l'alcool peut être comprise entre 1% et 10%, de préference entre 1% et 5%. La quantité de précurseur (II) à oxyder peut représenter entre 1 et 50% du poids total, de préference entre 5 et 20%. Enfin la température de réaction peut être comprise entre 20 et 100°C, de préference entre 70 et 90°C.

Les exemples suivants illustrent de façon non limitative la présente invention.

Exemples

Dans un réacteur de 250 ml équipé d'un agitateur à ancre tournant à 800 tours/minute, on place 150 ml d'une solution aqueuse de soude (NaOH) à 5%, 10 g de tétrahydro-1,4,4a,9a anthraquinone

ainsi que la quantité indiquée ci-dessous de produit P essayé comme antimousse. La suspension étant portée à 85°C, on insuffle de l'air à raison de 10 litres par heure au moyen d'un tube plongeant équipé à l'extrêmité d'un disque de verre fritté de porosité 2 et de 20 mm de diamètre.

Dans les procédés des exemples 1 à 6, la réaction est ainsi appliquée pendant deux heures; après quoi, on refroidit. L'anthraquinone en suspension est filtrée, lavée à l'eau et séchée. Dans les procédés des exemples contradictoires 7 à 12, il y a des phénomènes de moussage qui empêchent de poursuivre l'oxydation et l'anthraquinone n'est pas récupérée.

| N° exemple | P | Quantité P | Quantité de mousse | Rendement |
|---|---|---|---|---|
| 1 | éthanol | 4 g | Très peu de mousse | 98 % |
| 2 | alcool amylique primaire normal | 2 g | Pas de mousse | 98,5 % |
| 3 | ditto | 4 g | ditto | 99 % |
| 4 | ditto | 1 g | Un peu de mousse | 98 % |
| 5 | octanol primaire normal | 4 g | Pas de mousse | 99 % |
| 6 | butanol secondaire | 4 g | Pas de mousse | 98 % |
| 7 | néant | | Mousse très abondante débordement | |
| 8 | triéthylamine | 5 g | Mousse abondante | |
| 9 | xylène | 4 g | Mousse très abondante débordement | |
| 10 | butanol tertiaire | 5 g | Mousse très abondante | |
| 11 | mélange d'alcools oxo à 16 à 18 atomes de carbone | 1 g | Mousse très abondante | |
| 12 | ditto | 5 g | Mousse très abondante | |

**Revendications**

1. Procédé amélioré pour la préparation de l'anthraquinone par insufflation d'air dans une suspension de tétrahydro-1,4,4a,9a anthraquinone dans une solution aqueuse alcaline, caractérisé en ce que la suspension comprend un alcool primaire ou secondaire aliphatique monofonctionnel, linéaire ou ramifié, possédant 2 à 10 atomes de carbone.

2. Procédé selon la revendication 1 dans lequel la solution aqueuse alcaline est une solution aqueuse de soude, de potasse, de chaux ou de carbonate d'un métal alcalin à une concentration comprise entre 1% et la saturation.

3. Procédé selon la revendication 1 dans lequel la proportion en poids de tétrahydro-1,4,4a,9a anthraquinone est de 1 à 50%, plus spécialement de 5 à 20% par rapport au poids total du mélange réactionnel.

4. Procédé selon la revendication 1 dans lequel la concentration en poids de l'alcool ajouté pour éviter les phénomènes de moussage est comprise entre 1 et 10%, plus spécialement entre 1 et 5%, par rapport au poids total du mélange réactionnel.

5. Procédé selon la revendication 1 dans lequel l'alcool est l'éthanol.

6. Procédé selon la revendication 1 dans lequel l'alcool est le butanol normal ou secondaire.

7. Procédé selon la revendication 1 dans lequel l'alcool est un alcool amylique normal ou secondaire.

8. Procédé selon la revendication 1 dans lequel l'alcool est le n-octanol.

9. Procédé selon chacune des revendications 1 à 8 dans lequel la température de réaction est comprise entre 20°C et 100°C, préférentiellement entre 70°C et 90°C.

**Patentansprüche**

1. Verbessertes Verfahren zur Herstellung von Anthrachinon durch Einblasen von Luft in eine Suspension von 1.4.4a.9a-Tetrahydroanthrachinon in einer wässrigen alkalischen Lösung, dadurch gekennzeichnet, daß die Suspension einen linearen oder verzweigten primären oder sekundären aliphatischen Alkohol mit 2 bis 10 Kohlenstoffatomen enthält.

2. Verfahren nach Anspruch 1, bei dem die wässrige alkalische Lösung eine wässrige Lösung von Natrium-oder Kalium hydroxyd, Kalk oder eines Alkalimetallcarbonates in einer Konzentration zwischen 1% und dem Sättigungspunkt ist.

3. Verfahren nach Anspruch 1, bei dem das Gewichtsverhältnis von 1.4.4a.9a-Tetrahydroanthrachinon 1 bis 50%, vorzugsweise 5 bis 20%, bezogen auf das gesamte Reaktionsgemisch beträgt.

4. Verfahren nach Anspruch 1, bei dem die Gewichtskonzentration des zur Vermeidung des Schäumens zugefügten Alkohols zwischen 1 und 10%, vorzugsweise zwischen 1 und 5%, bezogen auf das Gesamtgewicht des Reaktionsgemisches beträgt.

5. Verfahren nach Anspruch 1, bei dem der Alkohol Äthanol ist.

6. Verfahren nach Anspruch 1, bei dem der Alkohol n- oder sec.-Butanol ist.

7. Verfahren nach Anspruch 1, bei dem der Alkohol ein n- oder sec.-Amylalkohol ist.

8. Verfahren nach Anspruch 1, bei dem der Alkohol n-Octanol ist.

9. Verfahren nach einem der Ansprüche 1 bis 8, bei dem die Reaktionstemperatur zwischen 20 und 100°C, vorzugsweise zwischen 70 und 90°C liegt.

**Claims**

1. Improved process for the preparation of anthraquinone by blowing air into a suspension of 1,4,4a,9a-tetrahydro-anthraquinone in an alkaline aqueous solution, characterised in that the suspension comprises a monofunctional aliphatic primary or secondary alcohol, linear or branched, containing 2 to 10 carbon atoms.

2. Process according to claim 1, in which the alkaline aqueous solution is an aqueous solution of sodium or potassium hydroxide, lime or the carbonate of an alkali metal, at a concentration between 1% and saturation.

3. Process according to claim 1, in which the proportion by weight of 1,4,4a,9a-tetrahydro-anthraquinone is from 1% to 50%, more especially from 5% to 20% with respect to the total weight of the reaction mixture.

4. Process according to claim 1 in which the concentration by weight of the alcohol added in order to prevent foaming is between 1% and 10%, more especially between 1% and 5%, with respect to the total weight of the reaction mixture.

5. Process according to claim 1, in which the alcohol is ethanol.

6. Process according to claim 1, in which the alcohol is normal or secondary butanol.

7. Process according to claim 1, in which the alcohol is a normal or secondary amyl alcohol.

8. Process according to claim 1, in which the alcohol is n-octanol.

9. Process according to each of the claims 1 to 8, in which the reaction temperature is between 20°C and 100°C, preferably between 70°C and 90°C.